# FASCICULE DE BREVET EUROPEEN

(11) **EP 3 911 391 B1**
(45) Date de publication et mention de la délivrance du brevet: **14.12.2022**
(21) Numéro de dépôt: 20705419.8
(22) Date de dépôt: 14.01.2020
(51) Int. Cl.: A61M 15/00, A61M 16/06, A61M 16/08, A61M 16/20

(54) **CHAMBRE D'INHALATION**
INHALATIONSKAMMER
INHALATION CHAMBER

(30) Priorité: 14.01.2019 FR 1900302
(43) Date de publication de la demande: 24.11.2021
(73) Titulaire: Stiplastics, 38160 Saint Marcellin (FR)
(72) Inventeur: BRICHET-BILLET, Etienne, 38470 NOTRE DAME DE L'OSIER (FR); ZOCZEK, Guillaume, 38210 TULLINS (FR); SOUAL, Ludovic, 26100 ROMANS SUR ISERE (FR); BROUARD, Hugues, 26300 ROCHEFORT SAMSON (FR)
(74) Mandataire: Regimbeau
(86) Numéro de dépôt international: PCT/FR2020/050041
(87) Numéro de publication internationale: WO 2020/148499

(56) Documents cités:
- FR-A1- 2 886 127
- FR-A1- 2 934 956
- GB-A- 2 468 108
- US-A- 6 014 972
- US-A1- 2002 069 870
- US-A1- 2007 283 954
- US-A1- 2016 256 641
- US-A1- 2018 236 189

## Description

### DOMAINE DE L'INVENTION

L'invention concerne une chambre d'inhalation.

### ETAT DE LA TECHNIQUE

Certains médicaments, par exemple des médicaments visant à traiter l'asthme, sont conçus pour être administrés sous la forme de microparticules dans les poumons du patient.

Le médicament est conditionné dans un aérosol doseur (également connu sous l'acronyme MDI, du terme anglo-saxon « Metered-Dose Inhaler») qui, sous l'effet d'une pression sur un actionneur exercée par un utilisateur (le patient lui-même ou un tiers, notamment dans le cas où le patient est un enfant), expulse une dose de médicament sous forme de microparticules à haute vitesse (de l'ordre de 100 km/h).

Du fait de la difficulté qu'il peut y avoir à parfaitement synchroniser actuation de l'aérosol doseur et aspiration buccale, et pour que le médicament ne se dépose pas dans la cavité buccale du patient mais parvienne jusqu'aux alvéoles pulmonaires, il est connu d'utiliser une chambre d'inhalation qui est un dispositif définissant un volume fermé comprenant un orifice d'entrée dans laquelle débouche l'embout de sortie de l'aérosol doseur et un orifice de sortie en liaison fluidique avec un embout buccal ou un masque appliqué sur le visage du patient. Les microparticules sont mises en suspension dans ce volume fermé, ce qui permet au patient de les inhaler en respirant simplement au travers de l'orifice de sortie.

Les chambres d'inhalation connues présentent une forme tubulaire, l'orifice d'entrée et l'orifice de sortie étant agencés aux deux extrémités du tube.

Cependant, la prise en main de la chambre d'inhalation et l'application de l'embout buccal ou du masque sur le visage du patient sont relativement malaisées.

En effet, du fait de la longueur du tube, il existe une distance importante entre l'aérosol doseur et le visage du patient. Le patient ou l'utilisateur doit donc tenir d'une main l'embout buccal et de l'autre main l'actionneur de l'aérosol doseur. Ceci est particulièrement malcommode lorsque le patient est un enfant ; en effet, il est souvent nécessaire que l'utilisateur place une main derrière la tête de l'enfant afin de le rassurer et/ou de maintenir sa tête pendant l'application du masque. L'autre main, qui manipule l'actionneur de l'aérosol est alors particulièrement éloignée de la première, ce qui rend difficile le bon maintien du masque sur le visage.

D'autre part, il est recommandé de maintenir l'aérosol doseur orienté verticalement, avec l'embout de sortie des microparticules situé en bas et l'extrémité de l'actionneur située en haut. On appelle dans la suite du texte cette position de l'aérosol « position verticale ».

Or, dans certains cas, cette orientation peut être difficile à conserver. Par exemple, lorsque le patient est couché, il n'est en général pas possible de maintenir l'aérosol vertical lors de l'administration du médicament.

Le document US 2018/0236189 décrit une chambre d'inhalation selon le préambule de la revendication 1. Les documents US 6,014,972 et FR 2 934 956 divulguent d'autres exemples de chambres d'inhalation.

### EXPOSE DE L'INVENTION

Un but de l'invention est de concevoir une chambre d'inhalation plus ergonomique et permettant d'utiliser l'aérosol doseur au mieux de la préconisation, à savoir en position verticale, quelle que soit la condition / position du patient.

A cet effet, l'invention propose une chambre d'inhalation comprenant un corps présentant un orifice d'entrée adapté pour être connecté à l'embout de sortie d'un aérosol doseur et un orifice de sortie adapté pour être connecté à un embout buccal ou un masque à appliquer sur le visage d'un patient, et un support adapté pour recevoir l'embout de sortie de l'aérosol doseur, ledit support débouchant dans l'orifice d'entrée, caractérisée en ce que l'axe de l'orifice d'entrée est non colinéaire à l'axe de l'orifice de sortie et en ce que ledit support est monté en pivotement sur le corps autour de l'axe de l'orifice d'entrée.

Selon un mode de réalisation préféré, l'axe de pivotement du support et l'axe de l'orifice de sortie sont orthogonaux.

De manière particulièrement avantageuse, le corps comprend deux coques démontables.

De préférence, au moins une desdites coques est transparente.

Selon un mode de réalisation, le support comprend une portion élastomérique adaptée pour assurer une liaison étanche entre l'embout de l'aérosol doseur et le support.

Selon un mode de réalisation, la chambre d'inhalation comprend en outre un capteur configuré pour détecter l'inclinaison de l'aérosol doseur.

Ledit capteur est avantageusement adapté pour être couplé à une unité de contrôle configurée pour comparer l'inclinaison mesurée avec une gamme d'inclinaison acceptable et émettre un signal selon le résultat de ladite comparaison à destination d'un indicateur lumineux, un avertisseur sonore et/ou un vibreur.

Selon une forme d'exécution, le corps comprend au moins un indicateur lumineux configuré pour s'éclairer différemment selon ladite comparaison.

De manière particulièrement avantageuse, le corps comprend en outre deux indicateurs lumineux en forme de flèche agencés de part et d'autre du support pour indiquer deux sens opposés de rotation dudit support, l'unité de contrôle étant configurée pour commander l'éclairage de l'un desdits indicateurs selon le sens de rotation à appliquer au support pour incliner l'aérosol doseur selon la gamme d'inclinaison acceptable.

La chambre d'inhalation peut en outre comprendre un masque monté en rotation sur le corps en liaison aéraulique avec l'orifice de sortie.

### BREVE DESCRIPTION DES FIGURES

D'autres caractéristiques et avantages de l'invention ressortiront de la description détaillée qui va suivre, en référence aux dessins annexés sur lesquels :
- la figure 1 est une vue en perspective d'une chambre d'inhalation selon un premier mode de réalisation ;
- la figure 2 est une autre vue en perspective de la chambre d'inhalation de la figure 1 ;
- la figure 3 est une vue de côté de la chambre d'inhalation de la figure 1 avec l'aérosol doseur en position verticale ;
- la figure 4 est une vue de côté de la chambre d'inhalation de la figure 1 avec une première autre orientation de la chambre d'inhalation, l'aérosol doseur étant toujours en position verticale ;
- la figure 5 est une vue de côté de la chambre d'inhalation de la figure 1 avec une seconde autre orientation de la chambre d'inhalation, l'aérosol doseur étant toujours en position verticale ;
- la figure 6 présente une vue en perspective et une vue de dessus d'une chambre d'inhalation selon un deuxième mode de réalisation ;
- la figure 7 présente une vue en perspective et une vue de dessus d'une chambre d'inhalation selon un troisième mode de réalisation ;
- la figure 8 présente une vue de côté d'un aérosol doseur.

Les signes de référence identiques d'une figure à l'autre désignent des éléments identiques ou tout au moins remplissant la même fonction.

### DESCRIPTION DETAILLEE DE MODES DE REALISATION DE L'INVENTION

Dans le présent texte sont mentionnés un utilisateur et un patient. L'utilisateur est une personne qui manipule la chambre d'inhalation et qui actionne l'aérosol doseur ; le patient est une personne qui inhale le médicament au travers de la chambre d'inhalation. Le patient et l'utilisateur peuvent naturellement être la même personne, notamment dans le cas d'un patient adulte apte à s'administrer une dose de médicament. En revanche, dans le cas d'un enfant ou d'un adulte qui n'est pas en capacité de manipuler lui-même la chambre d'inhalation, c'est un tiers qui manipule la chambre d'inhalation.

De manière connue en elle-même, l'aérosol doseur comprend un corps et un actionneur mobile en coulissement dans le corps en regard d'une valve doseuse. L'actionneur se présente sous la forme d'un réservoir contenant un gaz propulseur et une suspension micronisée du médicament. En poussant sur l'actionneur, un utilisateur libère une dose déterminée de médicament au travers de la valve doseuse. La valve doseuse débouche dans un embout de sortie du corps, l'axe de l'embout de sortie étant non colinéaire avec l'axe de coulissement de l'actionneur. De préférence, l'axe de coulissement de l'actionneur doit être maintenu vertical pendant l'utilisation de l'aérosol doseur.

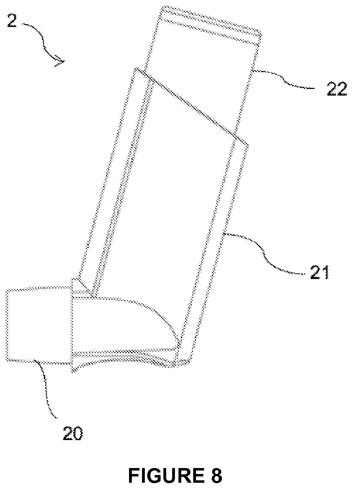

La figure 8 présente une vue de côté d'un tel aérosol doseur 2, comprenant un corps 21 et un actionneur 22, qui contient le gaz propulseur et les microparticules de médicament, monté en coulissement dans le corps 21. Le corps comprend un embout 20 de sortie des microparticules qui est incliné par rapport à l'axe de coulissement de l'actionneur 22.

Suivant le fabricant, la forme de l'aérosol doseur, notamment la forme et les dimensions de l'embout, est susceptible de varier. Cependant, de manière avantageuse, la chambre d'inhalation n'est pas limitée à un aérosol doseur spécifique mais, comme expliqué plus bas, peut recevoir différents aérosols doseurs du marché.

La chambre d'inhalation comprend un corps présentant un orifice d'entrée adapté pour être connecté à l'embout de sortie d'un aérosol doseur et un orifice de sortie adapté pour être connecté à un embout buccal ou un masque à appliquer sur le visage d'un patient.

Selon un mode de réalisation, l'embout buccal est solidaire du corps (il peut être par exemple moulé avec le corps) et le masque est adapté pour être monté de manière amovible sur le corps. Ainsi, le corps peut être utilisé soit par un patient enfant (avec le masque) soit par un patient adulte (directement avec l'embout buccal). Le masque comprend une valve de comptage au travers de laquelle passe l'air expiré par le patient.

L'orifice de sortie est pourvu d'une valve de volume qui évite tout retour aéraulique vers l'intérieur du corps. Une valve à bec de canard est particulièrement préférée du fait de sa capacité à s'ouvrir largement lors d'une aspiration sans effort excessif du patient, puis à se fermer de manière étanche, de sorte à éviter toute entrée d'air dans le corps lors d'une expiration.

La valve de volume et la valve de comptage peuvent être avantageusement réalisées en silicone de grade médical.

De manière avantageuse, le masque est monté pivotant sur l'embout buccal, de sorte à permettre d'ajuster l'orientation du masque par rapport au corps, notamment en fonction de la position du patient.

Le corps définit, entre l'orifice d'entrée et l'orifice de sortie, un volume fermé suffisamment grand pour permettre la mise en suspension des particules. La forme de ce volume fermé est définie en fonction notamment de l'emplacement des orifices d'entrée et de sortie pour minimiser la présence de zones dans lesquelles le médicament en suspension serait susceptible d'être retenu ou de se déposer.

L'aérosol doseur est monté sur le corps par l'intermédiaire d'un support qui débouche dans l'orifice d'entrée, l'embout de sortie de l'aérosol doseur étant en liaison aéraulique avec l'orifice d'entrée du corps pour permettre l'introduction des microparticules de l'aérosol doseur dans le corps.

Contrairement aux chambres d'inhalation connues, le support d'aérosol n'est pas fixe par rapport au corps, mais pivotant par rapport au corps, et ce, autour de l'axe de l'orifice d'entrée, qui n'est pas colinéaire avec l'axe de l'orifice de sortie. Par axe de l'orifice de sortie, on entend un axe perpendiculaire au plan formé par l'orifice de sortie.

La forme intérieure du support est avantageusement adaptable à la forme de l'embout de sortie de l'aérosol doseur, qui est généralement circulaire ou oblongue, de manière à assurer une étanchéité entre le support et l'embout. Selon un mode de réalisation préféré, le support comprend une portion élastomérique déformable selon la forme de l'embout de l'aérosol doseur, afin d'assurer à la fois l'étanchéité et la tenue mécanique de la liaison. Le support peut être réalisé par injection de deux matières plastiques différentes, une matière rigide assurant la liaison avec le corps et une matière élastomérique assurant la liaison avec l'embout de l'aérosol doseur.

L'axe de pivotement étant non colinéaire avec l'axe de l'orifice de sortie, il est possible par un simple pivotement du support de faire en sorte que l'aérosol doseur reste orienté verticalement, quelle que soit la position du patient.

Selon un mode de réalisation préféré, l'axe de pivotement est orthogonal à l'axe de l'orifice de sortie.

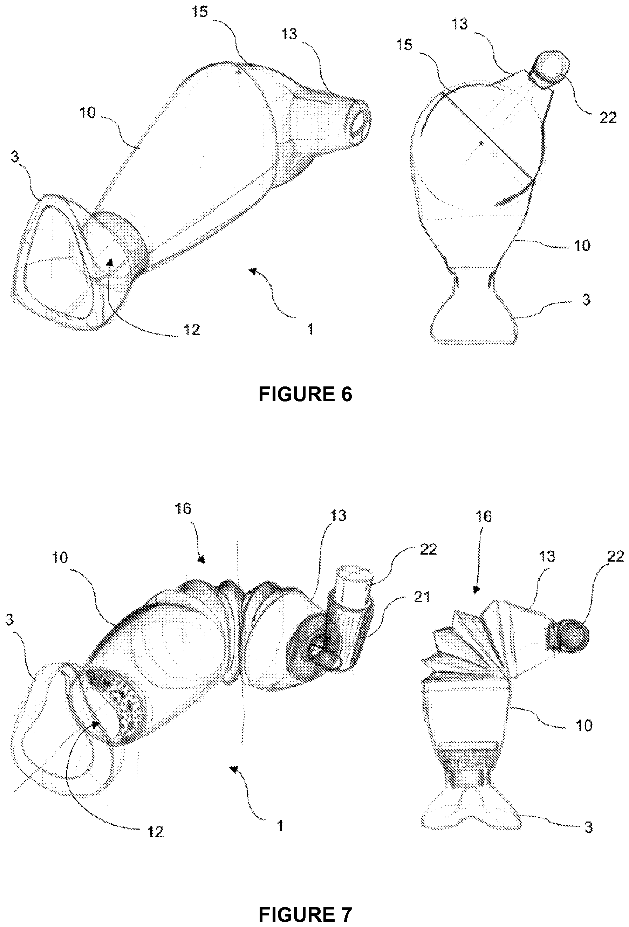

Le mouvement du support par rapport au corps peut ne pas être limité à une rotation autour d'un unique axe. Eventuellement, un degré de liberté supplémentaire en rotation peut être procuré par une liaison rotule (cf. figure 6) ou un soufflet (cf. figure 7).

En étant agencé sur un côté du corps, latéralement par rapport à l'embout buccal ou au masque, le support forme une poignée aisée à manipuler par l'utilisateur. La forme extérieure du support peut être définie pour améliorer l'ergonomie de la poignée.

En général, l'utilisateur tient la chambre d'inhalation au niveau du support/poignée par la main qu'il utilise préférentiellement dans ses tâches courantes (sa main droite s'il est droitier, sa main gauche s'il est gaucher). Le support peut donc être pivoté de sorte à pouvoir être tenu par la main droite ou la main gauche du patient, tout en conservant l'aérosol doseur vertical dans les deux cas.

Par ailleurs, la position du support par rapport à l'orifice de sortie peut être modifiée selon que l'utilisateur est le patient ou un tiers. En effet, dans ce dernier cas, l'utilisateur se place généralement face au patient, de sorte que la prise en main de la chambre d'inhalation est inversée.

Le pivotement du support permet de placer l'aérosol doseur du côté préféré pour l'utilisateur, qu'il soit le patient ou un tiers, tout en maintenant l'aérosol doseur orienté verticalement avec l'embout de sortie en partie inférieure.

Ledit support/poignée est également plus proche du visage du patient que dans les chambres d'inhalation de l'état de la technique. Il autorise ainsi une utilisation de la chambre d'inhalation à une main (l'utilisateur tenant la chambre d'inhalation par le support/poignée est à même d'appliquer correctement l'embout buccal ou le masque sur le visage du patient tout en actionnant l'actionneur). L'autre main reste donc libre, par exemple pour rassurer le patient lorsque celui-ci est un enfant.

Un autre avantage du positionnement du support de l'aérosol autour d'un axe non colinéaire à celui de l'axe de l'orifice de sortie est d'imposer au flux de microparticules une trajectoire selon une forme de L à l'intérieur du corps. Cette trajectoire a pour effet de ralentir les microparticules et de permettre aux microparticules les plus grosses (qui ne doivent pas être inhalées par le patient) de buter sur la face du corps opposée à l'orifice d'entrée et de tomber par gravité au fond du corps au lieu de sortir par l'orifice de sortie.

Le corps est fabriqué à partir de matériaux antistatiques. De manière particulièrement avantageuse, au moins l'un de ces matériaux est un matériau thermoplastique transparent, tel que de l'ABS (acrylonitrile butadiène styrène) par exemple. Ainsi, l'utilisateur peut visualiser les microparticules à l'intérieur du corps et observer le fonctionnement de la valve de volume.

Selon un mode de réalisation préféré, le corps est formé de deux coques assemblées de manière démontable par tout moyen adapté (par exemple par emboîtement, encliquetage, etc.) permettant un démontage manuel par l'utilisateur Ceci permet de nettoyer et sécher aisément l'intérieur du corps après utilisation de la chambre d'inhalation.

De manière avantageuse, lesdites coques sont assemblées selon un plan qui contient l'axe de pivotement du support de l'aérosol doseur. La coque qui contient l'orifice de sortie peut être réalisée en une matière antistatique opaque, tandis que la coque opposée peut être réalisée en une matière antistatique transparente. Il existe des grades d'ABS adaptés à ces deux fonctions.

Selon un mode de réalisation, le support de l'aérosol doseur est pourvu d'un capteur d'inclinaison adapté pour mesurer l'inclinaison de l'axe de coulissement de l'actionneur par rapport à la verticale. Ledit capteur est couplé à une unité de contrôle qui permet de comparer l'inclinaison mesurée à une gamme de valeurs d'inclinaison acceptables autour de la verticale.

L'unité de contrôle peut être couplée à un indicateur lumineux, un avertisseur sonore et/ou un vibreur, par exemple agencé(s)s sur le corps, activé(s) lorsque l'inclinaison mesurée est en-dehors de la gamme susmentionnée.

Selon un mode de réalisation, le corps comprend trois indicateurs lumineux, un indicateur principal étant configuré pour être éclairé avec une couleur verte si l'inclinaison de l'aérosol doseur est correcte, et avec une couleur rouge dans le cas d'une inclinaison incorrecte. Les deux autres indicateurs se présentent sous la forme de deux flèches agencées de part et d'autre du support et indiquant chacune un sens de rotation différent autour de l'axe de pivotement du support. Si l'indicateur principal est au rouge, l'une des flèches est éclairée pour indiquer dans quel sens faire tourner le support pour obtenir une inclinaison correcte de l'aérosol doseur, ce qui, une fois le geste effectué, fait passer au vert l'indicateur principal.

De manière alternative, l'unité de contrôle peut communiquer avec un dispositif électronique muni d'un écran, tel qu'un téléphone portable, de sorte à afficher sur l'écran l'inclinaison de l'aérosol doseur par rapport à la verticale. Des flèches peuvent également être affichées sur l'écran pour procurer à l'utilisateur des indications pour corriger une éventuelle inclinaison incorrecte.

Selon une forme d'exécution, la chambre d'inhalation comprend également un dispositif adapté pour mesurer la vitesse et/ou le débit d'air inspiré par le patient, de manière à pouvoir effectuer un suivi de l'amélioration ou la dégradation de la condition respiratoire du patient.

Par exemple, ledit dispositif peut comprendre un accessoire qui crée une cavité fermée autour du support d'aérosol doseur, dont l'entrée d'air est équipée d'un moyen de mesure de vitesse / débit d'air inhalé. Cette solution permet d'éviter d'entraver le parcours du médicament entre l'aérosol doseur et la bouche du patient.

Une solution similaire pourrait être utilisée pour mesurer la vitesse et/ou le débit d'expiration du patient, au-dessus de la valve de comptage.

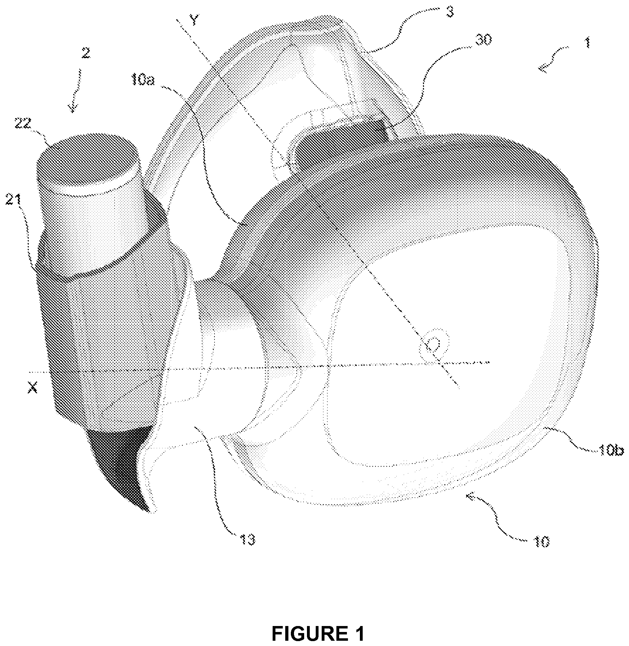

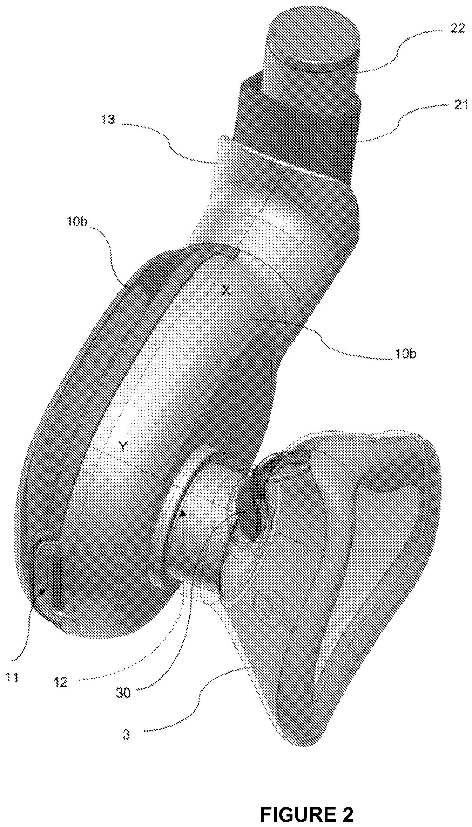

Les figures 1 à 5 illustrent un premier mode de réalisation de la chambre d'inhalation.

La chambre 1 comprend un corps 10, un support 13 adapté pour recevoir l'embout de sortie de l'aérosol doseur 2, débouchant dans le corps, et un masque 3 en liaison aéraulique avec un orifice 12 de sortie du corps. Les axes X et Y sont respectivement l'axe de pivotement du support 13 par rapport au corps et l'axe de l'orifice 12 de sortie du corps.

De manière connue en elle-même, une valve de volume (non visible sur les figures), par exemple du type à bec de canard, est agencée dans l'orifice de sortie. Le masque comprend par ailleurs une valve de comptage 30.

Du fait de la position du support par rapport au corps, lesdits axes ne sont pas colinéaires et sont avantageusement orthogonaux.

Le corps 10 comprend deux coques 10a, 10b assemblées selon un plan de joint qui contient l'axe de pivotement du support 13. De préférence, la coque 10a, qui comprend l'orifice de sortie, est en un matériau plastique opaque, tel que de l'ABS antistatique, tandis que la coque 10b est en un matériau plastique transparent, tel que de l'ABS antistatique. Les coques 10a, 10b comprennent sur leur périphérie des formes complémentaires permettant un emboîtement étanche et un maintien par friction, par exemple un système de fermeture par encliquetage 11. Elles peuvent donc être aisément séparées par un effort de traction de l'utilisateur dans une direction perpendiculaire au plan de joint en vue d'être nettoyées puis à nouveau assemblées.

Chacune des coques comprend la moitié d'un manchon cylindrique. Après assemblage des coques, le support 13 est emmanché en force sur ledit manchon. Les surfaces en vis-à-vis du manchon et du support sont conçues pour éviter un démontage intempestif du support 13 (tout en autorisant son démontage par application d'un effort suffisant par l'utilisateur) tout en autorisant une rotation du support autour du manchon. Ladite rotation peut être libre ou éventuellement indexée par un système d'encliquetage.

Le support 13 comprend sur sa face intérieure destinée à recevoir l'embout de l'aérosol doseur une portion élastomérique 14 qui permet d'assurer une liaison étanche avec l'embout tout en s'adaptant à la forme de celui-ci.

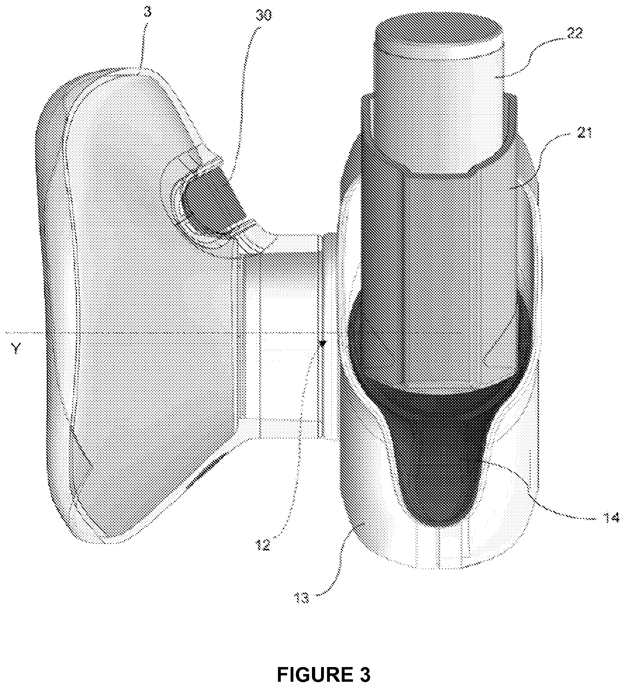

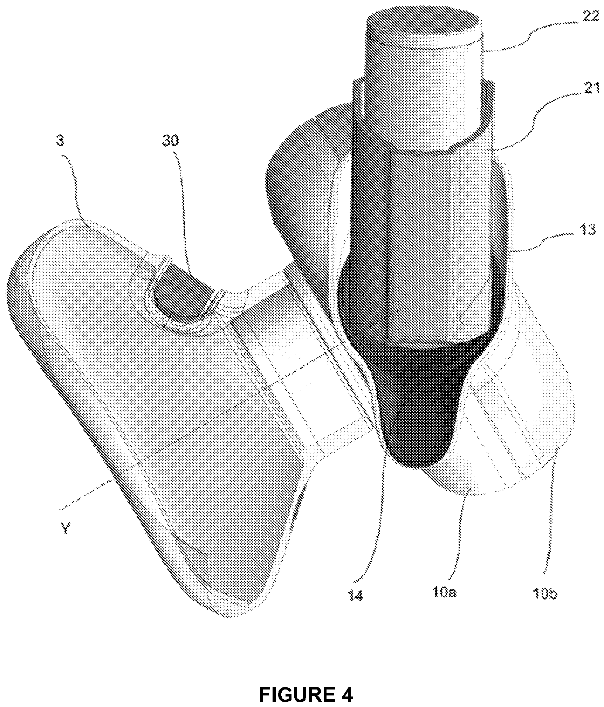

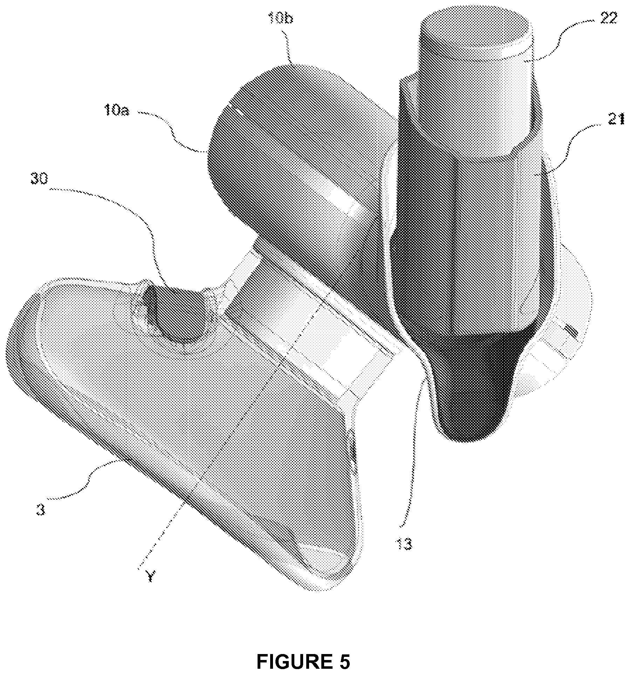

Sur la figure 3, la chambre d'inhalation est présentée dans une position adaptée à l'application sur un patient assis ou debout ; les figures 4 et 5 présentent la même chambre d'inhalation dans des positions inclinées adaptées à un patient semi-assis. Comme on peut le voir, le pivotement du support permet de conserver une orientation verticale de l'aérosol doseur. Sur les figures 3 à 6, l'axe de pivotement du support 13 est perpendiculaire au plan de la feuille.

La figure 6 illustre un deuxième mode de réalisation de la chambre d'inhalation.

Par rapport au mode de réalisation précédent, le corps 10 présente une forme allongée avec l'orifice de sortie 12 à une première extrémité et une rotule 15 à une seconde extrémité opposée à la première pour coupler le support 13 pour l'aérosol doseur au corps 10. Ladite rotule fournit donc un degré de liberté supplémentaire pour orienter le support par rapport au corps. Grâce à ce mécanisme, l'aérosol doseur peut être maintenu en position verticale quelle que soit la position du patient.

Dans cette configuration, le corps 10 peut être réalisé d'un seul tenant par moulage d'un matériau plastique antistatique, de préférence transparent. La rotule est ensuite insérée à la seconde extrémité du corps 10, de préférence de manière amovible afin de permettre le nettoyage et le séchage de la chambre d'inhalation.

Selon les applications, un masque 3 peut être monté sur la première extrémité du corps 10 de manière réversible.

La figure 7 illustre un troisième mode de réalisation de la chambre d'inhalation.

La construction du corps 10 est sensiblement identique à celle du deuxième mode de réalisation.

Dans cette variante, le support 13 de l'aérosol doseur est couplé au corps par l'intermédiaire d'un soufflet 16 qui peut être déformé selon au moins deux axes.

De manière particulièrement avantageuse, le soufflet 16 peut être démonté du corps afin de permettre le nettoyage et le séchage de la chambre d'inhalation.

## Revendications

1. Chambre d'inhalation (1) comprenant un corps (10) présentant un orifice d'entrée adapté pour être connecté à l'embout de sortie d'un aérosol doseur et un orifice de sortie (12) adapté pour être connecté à un embout buccal (30) ou un masque (3) à appliquer sur le visage d'un patient, et un support (13) adapté pour recevoir l'embout (20) de sortie de l'aérosol doseur (2), ledit support (13) débouchant dans l'orifice d'entrée, **caractérisée en ce que** l'axe (X) de l'orifice d'entrée est non colinéaire à l'axe (Y) de l'orifice de sortie et **en ce que** ledit support est monté en pivotement sur le corps (10) autour de l'axe (X) de l'orifice d'entrée.

2. Chambre d'inhalation selon la revendication 1, dans laquelle l'axe (X) de pivotement du support (13) et l'axe (Y) de l'orifice de sortie sont orthogonaux.

3. Chambre d'inhalation selon l'une des revendications 1 à 2, dans laquelle le corps (10) comprend deux coques démontables (10a, 10b).

4. Chambre d'inhalation selon la revendication 3, dans laquelle au moins une desdites coques est transparente.

5. Chambre d'inhalation selon l'une des revendications 1 à 4, dans laquelle le support (13) comprend une portion élastomérique (14) adaptée pour assurer une liaison étanche entre l'embout (20) de l'aérosol doseur et le support (13).

6. Chambre d'inhalation selon l'une des revendications 1 à 5, comprenant en outre un capteur configuré pour détecter l'inclinaison de l'aérosol doseur.

7. Chambre d'inhalation selon la revendication 6, dans laquelle ledit capteur est adapté pour être couplé à une unité de contrôle configurée pour comparer l'inclinaison mesurée avec une gamme d'inclinaison acceptable et émettre un signal selon le résultat de ladite comparaison à destination d'un indicateur lumineux, un avertisseur sonore et/ou un vibreur.

8. Chambre d'inhalation selon la revendication 7, dans laquelle le corps comprend au moins un indicateur lumineux configuré pour s'éclairer différemment selon ladite comparaison.

9. Chambre d'inhalation selon la revendication 8, dans laquelle le corps comprend en outre deux indicateurs lumineux en forme de flèche agencés de part et d'autre du support (13) pour indiquer deux sens opposés de rotation dudit support, l'unité de contrôle étant configurée pour commander l'éclairage de l'un desdits indicateurs selon le sens de rotation à appliquer au support pour incliner l'aérosol doseur selon la gamme d'inclinaison acceptable.

10. Chambre d'inhalation selon l'une des revendications 1 à 9, comprenant en outre un masque (3) monté en rotation sur le corps (10) en liaison aéraulique avec l'orifice de sortie.

## Patentansprüche

1. Inhalationskammer (1), umfassend einen Korpus (10), der eine Eingangsöffnung, die zum Anschließen an den Ausgangsansatz eines Dosieraerosols geeignet ist, und eine Ausgangsöffnung (12), die zum Anschließen an einen Mundansatz (30) oder eine Maske (3) geeignet ist, die auf das Gesicht eines Patienten angewendet ist, und einen Träger (13), der zum Aufnehmen des Ausgangsansatzes (20) des Dosieraerosols (2) geeignet ist, aufweist, wobei der genannte Träger (13) in die Eingangsöffnung mündet, **dadurch gekennzeichnet, dass** die Achse (X) der Eingangsöffnung zur Achse (Y) der Ausgangsöffnung nicht kollinear ist und dass der genannte Träger schwenkbar auf dem Korpus (10) um die Achse (X) der Eingangsöffnung angebracht ist.

2. Inhalationskammer gemäß Anspruch 1, bei der die Schwenkachse (X) des Trägers (13) und die Achse (Y) der Ausgangsöffnung orthogonal sind.

3. Inhalationskammer gemäß einem der Ansprüche 1 bis 2, bei der der Korpus (10) zwei abnehmbare Schalen (10a, 10b) umfasst.

4. Inhalationskammer gemäß Anspruch 3, bei der wenigstens eine der genannten Schalen transparent ist.

5. Inhalationskammer gemäß einem der Ansprüche 1 bis 4, bei der der Träger (13) einen elastomerischen Abschnitt (14) umfasst, der geeignet ist, um eine dichte Verbindung zwischen dem Ansatz (20) des Dosieraerosols und dem Träger (13) zu gewährleisten.

6. Inhalationskammer gemäß einem der Ansprüche 1 bis 5, umfassend darüber hinaus einen Sensor, der zum Detektieren der Neigung des Dosieraerosols ausgestaltet ist.

7. Inhalationskammer gemäß Anspruch 6, bei der der genannte Sensor geeignet ist, um an einer Kontrolleinheit gekoppelt zu sein, die zum Vergleichen der gemessenen Neigung mit einer akzeptablen Neigungsbandbreite und zum Aussenden eines Signals je nach dem Ergebnis des genannten Vergleichs an einen Lichtindikator, ein Signalhorn und/oder einen Vibrator ausgestaltet ist.

8. Inhalationskammer gemäß Anspruch 7, bei der der Korpus wenigstens eine Leuchtanzeige umfasst, die ausgestaltet ist, um je nach dem genannten Vergleich unterschiedlich aufzuleuchten.

9. Inhalationskammer gemäß Anspruch 8, bei der der Korpus darüber hinaus zwei Leuchtanzeigen in Pfeilform umfasst, die auf jeder Seite des Trägers (13) angeordnet sind, um zwei entgegengesetzte Drehrichtungen des genannten Trägers anzuzeigen, wobei die Kontrolleinheit ausgestaltet ist, um die Beleuchtung einer der genannten Anzeigen gemäß der Drehrichtung zu steuern, die auf den Träger anzuwenden ist, um das Dosieraerosol je nach der akzeptablen Neigungsbandbreite zu neigen.

10. Inhalationskammer gemäß einem der Ansprüche 1 bis 9, umfassend darüber hinaus eine Maske (3), die in Drehung auf dem Korpus (10) in lufttechnischer Verbindung mit der Ausgangsöffnung angebracht ist.

## Claims

1. An inhalation chamber (1) comprising a body (10) having an inlet port adapted to be connected to the outlet nozzle of a metered dose aerosol and an outlet port (12) adapted to be connected to a mouthpiece (30) or a mask (3) to be applied to the face of a patient and a support (13) adapted to receive the outlet nozzle (20) of the metered dose aerosol (2), said support (13) opening into the inlet orifice, **characterized in that** the axis (X) of the inlet orifice is non-collinear with the axis (Y) of the outlet orifice and **in that** said support is pivotally mounted on the body (10) about the axis (X) of the inlet orifice.

2. The inhalation chamber of claim 1, wherein the pivot axis (X) of the holder (13) and the axis (Y) of the outlet are orthogonal.

3. An inhalation chamber according to any of claims 1 to 2, wherein the body (10) comprises two removable shells (10a, 10b).

4. The inhalation chamber of claim 3, wherein at least one of said shells is transparent.

5. An inhalation chamber according to any of claims 1 to 4, wherein the holder (13) comprises an elastomeric portion (14) adapted to provide a tight connection between the mouthpiece (20) of the metered dose inhaler and the holder (13).

6. The inhalation chamber of any of claims 1 to 5, further comprising a sensor configured to detect the tilt of the metered dose aerosol.

7. The inhalation chamber of claim 6, wherein said sensor is adapted to be coupled to a control unit configured to compare the measured tilt with an acceptable tilt range and output a signal according to the result of said comparison to a light indicator, a buzzer and/or a vibrator.

8. The inhalation chamber of claim 7, wherein the body comprises at least one light indicator configured to illuminate differently according to said comparison.

9. The inhalation chamber of claim 8, wherein the body further comprises two arrow-shaped illuminated indicators arranged on either side of the holder (13) to indicate two opposite directions of rotation of said holder, the control unit being configured to control the illumination of one of said indicators according to the direction of rotation to be applied to the holder to tilt the metered dose aerosol within the acceptable range of inclination.

10. Inhalation chamber according to one of claims 1 to 9, further comprising a mask (3) rotatably mounted on the body (10) in aeroulic connection with the outlet port.
